# EUROPÄISCHE PATENTSCHRIFT

(11) **EP 1 763 535 B1**
(45) Veröffentlichungstag und Bekanntmachung des Hinweises auf die Patenterteilung: **23.01.2008**
(21) Anmeldenummer: 05761699.7
(22) Anmeldetag: 29.06.2005
(51) Int. Cl.: C07K 1/107

(54) **MIT EINEM ORGANISCHEM REST VERBUNDENES POLYPEPTID**
POLYPEPTIDE LINKED WITH AN ORGANIC GROUP
POLYPEPTIDE LIE A UN GROUPE ORGANIQUE

(30) Priorität: 29.06.2004 DE 102004031258
(43) Veröffentlichungstag der Anmeldung: 21.03.2007
(73) Patentinhaber: Jennissen, Herbert P., 45122 Essen (DE)
(72) Erfinder: Jennissen, Herbert P., 45122 Essen (DE)
(74) Vertreter: Viering, Hans-Martin
(86) Internationale Anmeldenummer: PCT/DE2005/001151
(87) Internationale Veröffentlichungsnummer: WO 2006/000209

(56) Entgegenhaltungen:
- EP-A- 0 242 656
- WO-A-88/03953
- WO-A-88/07076
- WO-A-92/10567
- MARUMO K ET AL: "OPTIMIZATION OF HYDROXYLATION OF TYROSINE AND TYROSINE-CONTAINING PEPTIDES BY MUSHROOM TYROSINASE" BIOCHIMICA ET BIOPHYSICA ACTA, AMSTERDAM, NL, Bd. 872, 1986, Seiten 98-103, XP000651113 ISSN: 0006-3002

## Beschreibung

Die Erfindung betrifft ein Verfahren zur Herstellung von einem mit einem organischen Rest modifizierten Polypeptid, sowie die so hergestellten modifizierten Polypeptide sowie deren Verwendung.

Aminosäure-Sequenzmotive am Ende eines Proteinhybrids werden auch als "Tag" (englisch für Etikett, Markierung, bindungsaktive Molekülgruppe) bezeichnet. Bisherige Konzepte zur Herstellung von solchen Protein-Tags gehen in der Regel von allgemeinen Affinitätsreaktionen (Enzym-Substrat, Effektor-Rezeptor, Biotin-Avidin oder Antigen-Antikörper Reaktionen) aus und nutzen die hohen Affinitäten des einen Partners gebunden an einer Oberfläche aus um den zweiten Partner zu binden. Eine besonders verbreitete Methode ist die His-Tag Methode, bei der ein Poly-Histidin-Schwanz an einem Fusionsprotein dazu dient, einen Chelatkomplex mit immobilisierten Metallionen z.B. Zn²⁺, Cu²⁺, Ni²⁺ (=MetallChelat-Affinitätstechnologie) auf einer Oberfläche zu bilden. Andere Konzepte und Techniken zur Bindung von Molekülen wie Proteinen auf Metalloberflächen beruhen beispielsweise darauf, eine nukleophile Gruppe mittels einer Silanisierungsreaktion auf der Metalloberfläche zu fixieren, die dann in einer zweiten Reaktion mit dem Protein zur Reaktion gebracht wird und über eine kovalente Bindung die beiden Moleküle verknüpft werden.

Nachteile der Silanisierungsverfahren sind darin zusehen, dass diese chemischen Reaktionen in wasserfreiem Milieu durchgeführt werden müssen und daher sehr arbeitsaufwendig und teuer sind. Außerdem führten diese drastischen Reaktionen häufig zur Denaturierung der immobilisierten Proteine.

Ein großer Nachteil der oben geschilderten Tag-Techniken ist der, dass nicht nur das Protein gentechnisch durch Anbringung beispielsweise eines His-Tags, sondern auch die den His-Tag bindende Oberfläche mit organischen chelatbildenden Molekülen modifiziert werden müssen, die die immobilisierten Zn²⁺, Cu²⁺oder Ni²⁺-Ionen tragen, um mit dem Poly-Histidinrest reagieren zu können.

Ein weiterer großer Nachteil der beschriebenen Methode ist, dass die ionentragende Oberfläche durch die relative niedrige Affinität der Chelatgruppe zu den immobilisierten Zn²⁺, Cu²⁺oder Ni²⁺-Ionen instabil ist und so zur Bindung des His-Tag-Proteins nur kurzzeitig genutzt werden kann.

WO 88103953, EP 0 242 656, WO 92/10567 und WO 88/07076 offenbaren Bioklebstoffe auf Basis von hydroxylierten aromatischen Peptiden, insbesondere Muschelpeptiden.

US 6,239,255 und EP 0489 465 offenbaren die Beschichtung von Goldoberflächen mit bioaktiven Verbindungen, die mittels Peptiden bzw. Thiolen an der Oberfläche befestigt werden.

Die Aufgabe der vorliegenden Erfindung besteht nun darin, Peptiden allein hochaffine Bindungseigenschaften gegenüber Metalloberflächen, Glas und Keramiken zu verleihen, ohne dass die Oberflächen dieser Materialien vorher modifiziert werden müssen.

Erfindungsgemäß lässt sich die oben dargestellte Aufgabe lösen durch die Bereitstellung eines mit einem organischen Rest modifizierten Polypeptid, bei dem ein bioaktives Polypeptid mit einem organischen Rest, der eine Rückgrat-Struktur mit aromatischen Seitenketten umfasst, kovalent verbunden wird und so ein modifiziertes Polypeptid aus bioaktivem Polypeptid und organischem Rest mit aromatischen Seitenketten gebildet wird, und zumindest eine aromatische Seitenkette des organischen Restes chemisch oder enzymatisch hydroxyliert wird.

Als organischer Rest sind erfindungsgemäß aus einzelnen Momomeren, die gleich oder unterschiedlich sein können, aufgebaute Reste verwendbar, die entweder direkt benachbart oder durch ein oder mehrere Monomere getrennt, aromatische Seitenketten aufweisen. Beispiele für solche organischen Reste sind solche mit Backbone-Strukturen (Rückgrat) aus C-Atomen wie Polymere von Ethylen, Propylen, Amiden, Ester-, Ether- oder Thioetherverbindung, die als aromatische Seitenketten Phenyl- oder Naphthylgruppen, Heterocyclen, aromatische Aminosäuren wie Phe, Tyr, oder Trp, etc aufweisen, die zusätzlich zumindest eine Hydroxylgruppe am aromatischen Rest tragen. Beispielhaft sind hier zumindest teilweise hydroxyliertes Polystyrol, oder Aminosäuresequenzen mit aromatischen hydroxylsubstituierten Aminosäuren genannt. Die Anzahl der Monomere kann dabei bis zu 50 betragen, wobei bevorzugt mehr als 5 Hydroxylgruppen im organischen Rest vorhanden sein sollen, um eine ausreichende Wechselwirkung mit der Oberfläche zu gewährleisten.

Aufgrund der vorhandenen Hydroxylgruppe an der aromatischen Seitenkette sind die Wechselwirkungen zwischen dem organischen Rest als Tag und der Werkstoffoberfläche von wesentlich höherer Stabilität als die beispielsweise eines His-Tags, so dass Langzeitbeschichtungen möglich sind. Das Spektrum der Nutzungsmöglichkeiten einer solchen organischen Tagtechnik stellt eine beträchtliche Erweiterung im Bereich des Tissue Engineering und der Biomaterialtechnologien dar.

Auf diese Weise ist es möglich, das Polypeptid, das - als mögliche bioaktive Mediatoren, Faktoren oder Gewebshormone - vorzugsweise Wachstumsfaktoren der TGF-β-Superfamilie wie TGF-β1 oder Knochenwachstumsfaktoren wie BMP-2, BMP-7, knorpelbildende Faktoren wie CDMP (GDF-5) oder Blutgefäßwachstumsfaktoren wie VEGF oder Angiotropin sowie PDGF und Nell-Proteine wie Nell-1 und Nell-2 umfasst, über diesen organischen Rest wie zuvor beschrieben auf der Substratoberfläche zu verankern und für weiter Anwendungen nutzbar zu machen.

Dabei kann der organische Rest endständig C-terminal oder N-terminal an das Polypeptid gebunden sein, oder der organische Rest mit aromatischen Seitenketten kann in das Polypeptid eingeschoben sein, solange die Aktivität des Polypeptides nicht nachteilig davon beeinflusst wird.

Besonders bevorzugt ist dabei, dass zumindest eine aromatische Seitenkette des organischen Restes chemisch oder enzymatisch so hydroxyliert wird, dass diese aromatische Seitenkette zwei Hydroxylgruppen trägt. So lässt sich eine besonders gute Wechselwirkung mit der Oberfläche eines Substrates erreichen, insbesondere bei solchen Substraten, die eine Oxy- oder Hydroxyguppen tragende Oberfläche aus Metalloxid, Metallhydroxid, Calciumhydroxyphosphaten (Hydroxyapatit), Siliciumoxid oder -hydroxid besitzen, wie sie bei Metallen, Keramiken oder Gläsern vorkommen.

In einen weiteren Ausführungsform des Verfahrens wird in einem ersten Schritt ein bioaktives Polypeptid mit einem organischen Rest, das eine Rückgrat-Struktur mit aromatischen Seitenketten umfasst, kovalent verbunden und ein modifiziertes Polypeptid aus bioaktivem Polypeptid und organischem Rest mit aromatischen Seitenketten gebildet, das die folgende Struktur aufweist:

P-[R₁-(X)ₙ]_{w}-[R₂-(X)ₙ]_{z}-R₃

wobei
P das bioaktive Polypeptid darstellt, das C-terminal oder N-terminal mit dem organischen Rest mit aromatischen Seitenketten verknüpft ist oder in das der organische Rest mit aromatischen Seitenketten eingeschoben ist;
R₁, R₂ und R₃ gleich oder unterschiedlich sind und jeweils für eine aromatische Aminosäure stehen, die aus Tyrosin, Tryptophan oder Phenylalanin ausgewählt wird;
X für eine beliebige Aminosäure, die innerhalb der Einheiten [R₁- (X)ₙ]_{w} und [R₂-(X)ₙ]_{z} gleich oder unterschiedlich ist;
n 0 bis einschließlich 10 ist;
w und z jeweils für eine natürliche Zahl von 0 bis 50 stehen; und
in einem zweiten Schritt zumindest eines von R₁, R₂ und R₃ so chemisch oder enzymatisch hydroxyliert wird, dass zumindest zwei Hydroxylgruppen am aromatischen Ring vorhanden sind.

Die natürlichen aromatischen Aminosäuren sind L-Phenylalanin (Phe), L-Tyrosin (Tyr) und L-Tryptophan (Trp). Da inzwischen auch das Vorkommen von D-Aminosäuren im Säugetier gesichert ist, sind auch D-Phenylalanin, D-Tyrosin und D-Tryptophan als Monomere für solche Reste denkbar. Gentechnisch herstellbare organischen Aminosäuresequenzen, die erfindungsgemäß verwendet werden können, können aus n = 2-50 aromatischen Aminosäuresequenzen wie - (Phe)ₙ -, - (Tyr)ₙ -, - (Trp)ₙ - oder Kombinationen davon, die entweder am N- oder C-Terminus eines Zielpolypeptides gebunden sind. Diese Aminosäuresequenzen können als X eine beliebige Aminosäure enthalten, die innerhalb der Einheiten [R₁-(X)ₙ]_{w} und [R₂-(X)ₙ]_{z} gleich oder unterschiedlich ist. Dabei sind auch solche Analoga eingeschlossen, bei denen die Stereochemie der einzelner Aminosäuren von L/S zu D/R an einer oder mehrerer spezifischen Positionen geändert ist. Analoga sind ebenfalls eingeschlossen, die weniger Peptid-Charakter haben. Solche Peptid-Mimetics können beispielhaft eine oder mehrere der Gruppen der folgenden Substitutionen für CO-NH-Amid-Bindungen haben: Depsipeptide (CO-O); Iminomethylene (CH2-NH); trans-Alkene (CH=CH), Enaminonitrile (C(= CH-CN)-NH), Thioamide (CS-NH), Thiomethylene (S-CH2), Methylene (CH2-CH2) and Retro-amide (NH-CO), die beispielsweise die Stabilität des organischen Restes P-[R₁-(X)ₙ]_{w}-[R₂-(X)ₙ]_{z}-R₃ gegenüber Proteasen im physiologischen Milieu erhöht. Diese Substitutionen können in dem erfindungsgemäß verwendeten organischen Rest überall dort verwendet werden, wo Peptidbindungen vorliegen.

Die Hydroxylierung der aromatischen Seitenketten kann über bekannte chemische Verfahren oder enzymatisch erfolgen. Bevorzugt ist daher in dem erfindungsgemäßen Verfahren zumindest ein aromatischer Rest davon chemisch oder enzymatisch so hydroxyliert, dass zwei Hydroxylgruppen benachbart am aromatischen Ring, mehr bevorzugt drei Hydroxylgruppen benachbart am aromatischen Ring vorhanden sind.

Dabei kann die Synthese des organischen Rests auf chemischem Wege erfolgen und dieser organische Rest an einem Aminosäurerest des bioaktiven Polypeptids kovalent gebunden werden. Alternativ dazu kann gentechnisch ein mit einem organischen Rest modifiziertes Polypeptid in Pro- oder Eukaryontenzellen synthetisiert werden.

In einer Ausführungsform des erfindungsgemäßen Verfahrens ist in der obigen Formel P-[R₁-(X)ₙ]_{w}-[R₂-(X)ₙ]_{z}-R₃ n kleiner als drei, bevorzugt gleich 0 oder 1 und w und Z sind jeweils eine ganze Zahl von 0 bis 5.

Die vorliegende Erfindung ist daher auch gerichtet auf ein mit einem organischen Rest modifizierten Polypeptid, das aus bioaktivem Polypeptid und organischem Rest mit aromatischen Seitenketten gebildet wird, wobei zumindest eine aromatische Seitenkette des organischen Restes chemisch oder enzymatisch hydroxyliert ist.

Dabei hat der organische Rest im Peptid bevorzugt zumindest in einer aromatischen Seitenkette zwei Hydroxylgruppen.

Weiter betrifft die Erfindung auch ein Polypeptid mit der folgenden Struktur:

P-[R₁-(X)ₙ]_{w}-[R₂-(X)ₙ]_{z}-R₃

wobei
P das bioaktive Polypeptid darstellt, das C-terminal oder N-terminal mit dem Rest mit aromatischen Seitenketten verknüpft ist oder in das der organische Rest mit aromatischen Seitenketten eingeschoben ist;
R₁, R₂ und R₃ gleich oder unterschiedlich sind und jeweils für eine aromatische Aminosäure stehen, die aus Tyrosin, Tryptophan oder Phenylalanin ausgewählt wird;
X für eine beliebige Aminosäure steht, die innerhalb der Einheiten [R₁-(X)ₙ]_{w} und [R₂- (X)ₙ]_{z} gleich oder unterschiedlich ist;
n 0 bis einschließlich 10 ist;
w und z jeweils für eine natürliche Zahl von 0 bis 50 stehen;
wobei zumindest eines von R₁, R₂ und R₃ so chemisch oder enzymatisch hydroxyliert wird, dass zumindest zwei Hydroxylgruppen am aromatischen Ring vorhanden sind.

Von besonderer Bedeutung als organische Reste sind dabei Poly-Phe, Poly-Tyr, Poly-Trp, die auf Grund ihres aromatischen Charakters direkt über π-π oder d-π Donor-Akzeptor Wechselwirkungen mit entsprechenden π- oder d-Elektronen-haltigen Verbindungen beispielsweise auf einer metallischen Oberfläche in Wechselwirkung treten können. Ferner können sie nach Einführung von Hydroxylgruppen über Oxidationsverfahren in entsprechende Hydroxyverbindungen umgewandelt werden. Tyrosin ist beispielsweise eine natürliche aromatische Hydroxyverbindung. Durch Einführung einer weiteren Hydroxylgruppe in das Tyrosin wird beispielsweise Dihydroxyphenylalanin (DOPA) und aus einem entsprechenden Poly-Tyr (-(Tyr)ₙ-) ein Poly-DOPA (-(DOPA)ₙ-) gebildet.

So kann mittels eines Verfahrens zur Beschichtung eines Substrates eine Lösung des Polypeptids auf die Oberfläche eines Substrates aufgebracht wird und das Polypeptid mittels kovalenter oder nicht-kovalenter Wechselwirkungen auf der Substratoberfläche immobilisiert werden. Das Substrat kann besonders Metall, Keramik oder Glas sein und eine Oxy- oder Hydroxygruppen tragende Oberfläche aus Metalloxid, Metallhydroxid, Calciumhydroxyphosphaten (Hydroxyapatit), Siliciumoxid oder -hydroxid besitzen.

Im Falle des Poly-Tyr ist der Poly-Tyrosin-Tag bereits nach dem ersten Schritt als polyphenolische Gruppe vorhanden und ist direkt für eine Bindungsreaktion z.B. auf Metalloberflächen einsetzbar. Es ist jedoch zu erwarten, dass die Einführung einer zweiten phenolischen Hydroxylgruppe in das Tyrosin zu einer Erhöhung der Spezifität der Bindung und der Affinität (=Bindungsenergie) führt. Daher können in einem zweiten Schritt eine oder mehrere phenolische Hydroxylgruppen in das aromatische Ringsystem des Phenylalanins, des Tyrosins oder des Tryptophans eingeführt werden. Beispielweise die Aminosäure Tyrosin (4-Hydroxy-Phenylalanin) kann auf diesem Wege in 3,4-Dihydroxy-phenylalanin (DOPA) überführt werden. So kann aus einem Poly-Tyrosin-Tag ein Poly-DOPA-Tag hergestellt werden. Eine weitere Hydroxylierung zu 3,4,5-Trihydroxyphenylalanin (TOPA) ist ebenfalls möglich. Der polyphenolische Tag z.B. Poly-DOPA-Tag kann dann den Proteinen besondere Haftungseigenschaften auf Metalloberflächen, insbes. Übergangsmetallen, Glasoberflächen oder Keramiken verleihen, so dass eine dauerhafte Beschichtung der Werkstoffoberflächen für vielfältige biologische, chemische und medizinische Anwendungen ermöglicht wird.

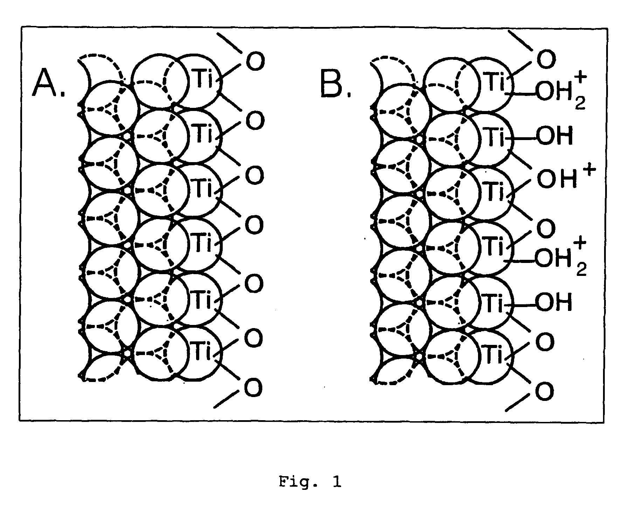

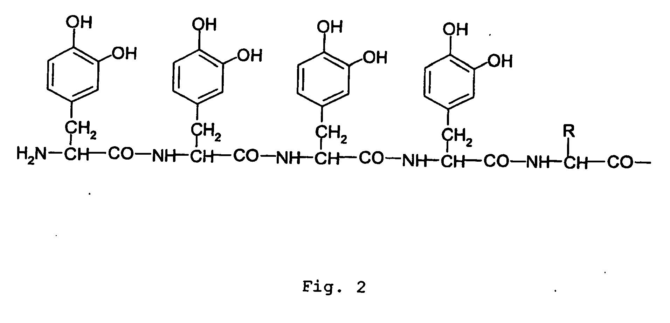

Polyphenolische Tags wie z.B. Poly-DOPA können spezifische Bindungsreaktionen mit bestimmten Übergangsmetalloxiden auf Metalloberflächen eingehen. Folgende chemische Reaktionsweisen für die Bindung eines Proteins über einen Poly-DOPA Tag an einer Titanoberfläche sind denkbar:
1. Ionische Wechselwirkung zwischen positiven Ladungen auf der Titanoberfläche (Abb. 1B) und negativ geladenen Phenolat-Ionen des Poly-DOPA (Abb. 2).
2. Elektron-Donor-Akzeptor Komplexe in Form einer d-π Wechselwirkung zwischen dem Titan (d-Orbitale) und den π-Elektronen des Phenolringes.
3. Möglicherweise kann auch eine direkte metallorganische Verbindung zwischen DOPA und der Titanoberfläche eingegangen werden.

Die besonderen Haftungseigenschaften beispielsweise von Poly-DOPA-Tags an Fusionsproteinen werden in dem Verfahren gemäß dieser Erfindung genutzt, um Proteine selektiv und mit hoher Affinität an Metall- oder Glasoberflächen direkt zu immobilisieren. Vermutlich sind die Hydroxylgruppen in Orthostellung am Phenylrest (d.h. DOPA) in der Struktur (DOPA)₃ verantwortlich für die hochaffine Bindung von Rest-DOPA an den Hydroxylgruppen einer Titandioxydoberfläche, wie sie auf metallischem Titan vorkommen, wie es in Fig. 1 gezeigt wird. Damit wurde die Möglichkeit 3 (s.o.) voll bestätigt. Die Möglichkeiten 1 und 2 sind jedoch dadurch nicht ausgeschlossen, sondern können zusätzlich wirken.

Durch die gleichzeitige Reaktion mehrerer DOPA-Reste in einem Poly-DOPA-Molekül mit der Titanoberfläche wird die Affinität der Bindung in einer Potenzfunktion zunehmen, so daß extrem hohe Bindungsaffinitäten (10⁸-10¹⁵ M⁻¹) erreicht werden können. Übergangsmetalloxidhaltige Oberflächen können somit zu Trägermaterialien für organische Reste tragende Proteine werden und zur Synthese von biologisch aktiven Oberflächen im Bereich Tissue-Engineering und Biomaterialien-Engineering eingesetzt werden. Die Anwendung dieser Technologie ist auch auf Glasoberflächen möglich. So können Matrices für natürliche, rekombinante oder synthetische Proteine oder Peptide erstellt werden, die sich auch im Bereich der Chromatographie, der Immunoassays und der Arraytechnik bewähren.

Als mögliche bioaktive Peptide wie Mediatoren, Faktoren oder Gewebshormone können vorzugsweise Wachstumsfaktoren der TGF-β-Superfamilie wie TGF-β1 oder Knochenwachstumsfaktoren wie BMP-2, BMP-7, knorpelbildende Faktoren wie CDMP (GDF-5) oder Blutgefäßwachstumsfaktoren wie VEGF oder Angiotropin sowie PDGF sowie Nell-Proteine wie Nell-1, Nell-2 verwendet werden.

Die einfach oder mehrfach hydroxylierten aromatischen Polyaminosäuren in kovalenter Bindung mit einem definierten Zielprotein wie einem Enzym, Wachstumsfaktor (s.o.) oder einem Strukturprotein dienen als Ankerstruktur für die feste Bindung des Zielproteins an eine siliziumoxid- oder metalloxidhaltige Matrix. Durch diese Bindung an die oxidhaltige Matrix kann das Fusionsprotein aus einem Extrakt gereinigt werden oder auf einer Metalloberfläche beispielsweise eines Titanimplantates in biologisch aktiver Form immobilisiert werden.

Die Synthese eines polyphenolischen Tags wird im Folgenden hier am Bespiel des Poly-L-Tyrosins und des Poly-L-DOPAs beispielhaft beschrieben. Analoge Methoden können für Poly-Phenylalanin- und Poly-Tryptophan-Tags erstellt werden. Es wird ein Verfahren beschrieben, das in wässrigem Milieu und unter schonenden Bedingungen durchgeführt werden kann. Das gewünschte Fusionsprotein, bei dem der N- oder C-Terminus frei, d.h. nicht im Inneren des Proteins versteckt vorkommen muss, wird in folgenden drei Schritten hergestellt:

Die Triplettcodes für Tyrosin lauten UAU und UAC. Somit führen (UAU) ₙ und (UAC)ₙ in Fusion mit einem Zielprotein am C-Terminus zu Protein-(Tyr)ₙ (Präkursorpolypeptid):

(1) cDNA- (UAC)ₙ → Protein-C-Term-(Tyr)ₙ

(2) (UAC)ₙ-cDNA → (Tyr)ₙ-N-Term-Protein

Die Anzahl der Tyrosinreste liegt vorzugsweise zwischen 3-5 wobei die Tyrosinreste entweder wie in Gl. 1 und 2 direkt aufeinander folgen in Form eines Homopolymers oder aber durch andere Aminosäuren getrennt sein können (Heteropolymer) beispielsweise in der Formel P-[Tyr-(X)ₙ]_{w}-[Tyr-(X)ₙ]_{z}-Tyr, wobei X eine beliebige Aminosäure in beliebiger Sequenz sein können und n vorzugsweise zwischen 1 bis 5 liegen kann. Da Tyrosin und Polytyrosin in Wasser schwerlöslich sind, sind saure oder basische Aminosäuren als X in der Formel P-[Tyr-(X)ₙ]_{w}-[Tyr-(X)ₙ]_{z}-Tyr besonders günstig, wenn die Löslichkeit des Fusionsproteins durch den Poly-Tyr Tag nicht gesenkt werden soll. Von besonderem Vorteil kann es sein, das Tyrosin in bestimmten definierten Abständen im Polypeptid vorkommen zu lassen, damit die Hydroxylgruppen sich der Oberflächentopographie der Hydroxylgruppen des Metalloxids anpassen können. Das z.B. in E. coli oder in CHO-Zellen (Chinese Hamster Ovary Cells) gentechnisch so hergestellte Protein muss dann zunächst angereichert und gereinigt werden. Hierfür kann man einen Doppeltag bestehend aus einem N-terminalen Poly-Tyr Tag und einem C-terminalen Poly-His Tag zur Aufreinigung verwenden. Alternativ könnte man den Poly-Tyr-Tag mit einem Poly-His Tag kombinieren und evtl. später durch bekannte Verfahren den Poly-His Tag abspalten. Oder aber das Protein, z.B. rhBMP-2, wird nach klassischen Methoden gereinigt.

Im nächsten Schritt wird die aromatische Aminosäure Phe, Tyr, DOPA, hydroxyliert. Im Falle von Tyrosin kann dies entweder enzymatisch oder chemisch erfolgen.

Peptidyltyrosinhydroxylase oder die Pilztyrosinase (Tyrosinase) mit Sauerstoff und einem Reduktionsmittel NADH + H⁺ oder Ascorbinsäure ergibt dann

(3) Protein-(Tyr)ₙ → Protein-C-Term-(DOPA)n

Umgekehrt führen (UAU) ₙ und (UAC)ₙ in Fusion mit einem Zielprotein am N-Terminus unter ähnlichen Bedingungen zu:

(4) (Tyr)ₙ-N-Term-Protein → (DOPA)ₙ-N-Term-Protein

Auch hier können, wie bereits oben dargestellt wurde, Homo-oder Heteropolymere des Präkursor-Tyrosinpeptids vorliegen, die dann in entsprechende Homo- oder Heteropolymere des DOPA umgewandelt werden. Hier könnte es von besonderem Vorteil sein, dass die DOPA-Moleküle in dem Polypeptid einen definierten Abstand erhalten, der besonderen sterischen Verhältnissen der Metalloxydschicht der Metalloberfläche entspricht. Auf ähnliche Weise ließen sich entsprechende polyorganischen Aminosäurenhybride aus den Aminosäuren Phenylalanin und Tryptophan erstellen.

In einer dritten Möglichkeit können auch [R₁-(X)ₙ]_{w}-[R₂-(X)ₙ]_{z}-R₃ wie beispielsweise Poly-X-Sequenzen mitten in Proteine eingeschoben werden, sofern die biologische Aktivität dies erlaubt. Man kann sich eine solche Anwendung vorstellen, für den Fall, dass der N-Terminus oder der C-Terminus nicht modifiziert werden dürfen. Man kann sich eine solche Anwendung vorstellen, für den Fall, dass der N-Terminus oder der C-Terminus aus Aktivitätsgründen nicht modifiziert werden dürfen oder der Terminus nicht frei sondern im Inneren des Proteins vorliegt.

Die in den dargestellten Verfahren hergestellten Fusionsproteine können dann über das polyorganische Aminosäure-Hybrid an Metall-, Keramik- oder Glasoberflächen gebunden werden. In einer besonderen hier als Beispiel angegebenen Anwendungsform mit dem Knochenwachstumsfaktor BMP-2 (bone morphogenetic protein 2) kann ein am N-Terminus fusionierter Poly-DOPA- oder Poly-TOPA-Tag dazu verwendet werden, BMP-2 in biologisch aktiver Form allein durch Inkubation mit einem Titanimplantat an der Titanoberfläche hochaffin zu binden und als bioaktives Zahn-, Hüft- oder Knieimplantat am Menschen einzusetzen. Mit einer hohen Bioaktivität des BMP-2 ist daher zu rechnen, dass N-terminale Peptidverlängerungen nach Art eines Poly-DOPA-Tags mit 5-10 Aminosäuren in der Regel nicht zu Einbußen in der biologischen Aktivität führen. Darüber hinaus führt eine Bindung des BMP-2 über einen N-terminalen Poly-DOPA-Tag zu einer orientierten Immobilisierung des BMP-2 auf der Titanoberfläche führen und damit eine außerordentlich hohe spezifische biologische Aktivität bedingen. Alternativ könnten beispielsweise Proteine mit einem Poly-DOPA Tag an Glasmikrokugeln oder Silikagelkugeln gebunden werden und als Affinitätsliganden in der Affinitätschromatographie eingesetzt werden.

Die Erfindung wird anhand der folgenden Figuren uns Beispiele weiter erläutert. Dabei zeigen
Figur 1 das Strukturmodell der Titandioxidoberfläche eines Titanwerkstoffes als Modell einer Oxidschicht der Übergangsmetalle oder einer Glasoberfläche; und
Figur 2 die Struktur eines N-terminal lokalisierten Poly-3,4 Dihydroxyphenylalanin-Tags (= Poly-DOPA-Tag) an einem hypothetischen Fusionsprotein, beispielsweise der Familie TGF-β.

Wie in Figur 1 gezeigt, zeigt Teilabbildung A und B eine nicht-hydrolysierte Oxidschicht (A) und eine hydrolysierte Oxidschicht mit protonierten Gruppen (B). Der Isoelektrische Punkt liegt etwa bei pH 4.5. Die Reaktionsweisen der Hydroxylgruppen der hydrolysierten Oxidschicht ist wie folgt:

Terminale Hydroxylgruppe: ≡ TiOH⁺₂ - H⁺ ⇆ ≡TiOH - H⁺ ⇆ ≡TiO⁻ (1)

Brückenhydroxylgruppe: ≡ Ti-OH⁺-Ti ≡ - H⁺ ⇆ ≡ Ti-O-Ti ≡ (2)

Wie in Fig 2. gezeigt, ist die Struktur eines N-terminal lokalisierten Poly-3,4 Dihydroxyphenylalanin-Tags (= Poly-DOPA-Tag) an einem hypothetischen nicht dargestellten Polypeptid, beispielsweise der Familie TGF-β angegeben. Dabei können die Phenolatgruppen des Poly-DOPA in ein Proton und das entsprechende negativ geladene Phenolation dissoziieren (pK - 10.0).

### Herstellungsbeispiele:

### Beispiel 1 - Hydroxylierung von Tyrosin-enthaltenden Peptiden

Phosphat-Borat-Ascorbat-Puffer:
0.1 M Phosphatpuffer
0.02 M Borat
Einstellung des pH-Wertes mit Ascorbinsäure auf pH 7.0

Präparativer Ansatz für Modellpeptide:
10 ml Phosphat-Borat-Ascorbat-Puffer, pH 7.0
2 mg Mushroom Tyrosinase (Fa. Sigma, 6680 U/mg)
10 mg Tyrosin-Peptid (Tyr-Tyr-Lys-His-Lys-Tyr-Tyr oder Ala-Lys-Pro-Ser-Tyr-Pro-Pro-Thr-Tyr-Lys)
Inkubation für 40 Minuten (20-30 °C) unter Rühren (oder Durchperlung mit Sauerstoff)

Ausbeute an DOPA-haltigem Peptid: ~ 80%

### Beispiel 2 - Hydroxylierung eines rhBMP-2 mit einem N-terminalen Poly-Tyr Tag (3-5 Tyr)

Für die Hydroxylierung von rhBMP-2 muss ein anderes Puffersystem verwendet werden als in Beispiel 1 bei den künstlichen Peptiden, da das rhBMP-2 bei pH 7.0 schlecht löslich ist.

Boratpuffer:
0.125 NaBorat, pH 9-10
0.066% Natriumdodecylsulfat
25 mM Ascorbinsäure

Präparativer Ansatz für BMP-2:
10 ml Borat-Ascorbat puffer, pH 9-10
2 mg Mushroom Tyrosinase (Fa. Sigma, 6680 U/mg)
2 mg rhBMP-2 mit Poly-Tyr Tag (n = 3-5)
Inkubation für 30-40 Minuten (20-30 °C) unter Rühren (oder Durchperlung mit Sauerstoff)

Die Hydroxylierungsreaktion läuft dabei bei einem pH von 9.0 wesentlich schneller ab als bei pH 7.0. Das ist hier von Vorteil, da das rhBMP-2 bei einem pH von 7.0 praktisch unlöslich, dagegen in dem o.g. Boratpuffer, pH 9-10 sehr gut löslich ist.

Obgleich zu erwarten ist, dass die Hydroxylierung des Poly-Tyr Tags am Poly-Tyr-rhBMP-2 auch Tyrosinreste im Molekülverband des BMP-2 selbst hydroxyliert, hat nach Erkenntnissen des Erfinders keinen Einfluss auf die biologische Aktivität des rhBMP-2 haben. So haben Iodierungsversuche, wobei mit ¹²⁵I nach der Chloramin T Methode Tyrosinreste im rhBMP-2 Molekül iodiert werden, gezeigt, dass die biologische Aktivität des rhBMP-2 jedoch voll erhalten bleibt. Daraus kann man schließen, dass eine Modifikation der Tyrosinreste durch Einführung einer zweiten Hydroxylgruppe ebenfalls nicht zu einer Beeinträchtigung der biologischen Aktivität führt. Man kann schließen, dass vermutlich keine Tyrosinreste direkt an der biologischen Aktivität des rhBMP-2 beteiligt sind.

Im Falles des BMP-2 führt auch die Modifikation des N-terminalen Endes nicht zu einem Aktivitätsverlust des BMP-2. Es wurde bereits gezeigt, dass die 12 N-terminalen Aminosäuren des rhBMP-2 durch ein Fremdpeptid mit 17 Aminosäuren (d.h. es ist 5 Aminosäuren länger!) ohne Verminderung der biologischen Aktivität ersetzt werden kann. Es sollte also problemlos möglich sein, gentechnisch ein Pentapeptid mit 3-5 Tyrosinresten an den N-Terminus des rhBMP-2 zu fusionieren, ohne dass eine Beeinträchtigung der Aktivität eintritt. Es konnte ferner anhand der Röntgenstruktur des rhBMP-2 gezeigt werden, dass der N-Terminus des rhBMP-2 frei beweglich vorkommt. Der N-Terminus stellt sich nämlich wegen der freien Beweglichkeit in der Röntgenstrukturanalyse nicht dar.

## Patentansprüche

1. Ein mit einem organischen Rest modifiziertes Polypeptid,
**dadurch gekennzeichnet, dass**
das modifizierte Polypeptid aus einem bioaktiven Polypeptid, das kovalent mit einem organischen Rest, der eine Rückgrat-Struktur mit aromatischen Seitenketten umfasst, verbunden ist, gebildet wird,
wobei der organische Rest, der eine Rückgrat-Struktur mit aromatischen Seitenketten umfasst, aus der Gruppe, die aus
(i) Polymeren von bis zu 50 Monomeren von Ethylen, Propylen, Ester-, Ether- oder Thioetherverbindungen, die als Seitenketten Phenyl- oder Naphtylgruppen, Heterocyclen oder aromatische Aminosäuren wie Phe, Tyr oder Trp aufweisen, die zumindest eine Hydroxylgruppe am aromatischen Rest tragen und wobei zumindest eine aromatische Seitenkette des organischen Restes chemisch oder enzymatisch so hydroxyliert wird, dass diese aromatische Seitenkette zwei Hydroxylgruppen trägt;
(ii) einem Rest mit der Struktur
P-[R₁-(X)ₙ]_{w}-[R₂-(X)n]_{z}-R₃
wobei
P das bioaktive Polypeptid darstellt, das C-terminal oder N-terminal mit dem organischen Rest mit aromatischen Seitenketten verknüpft ist oder in das der organische Rest mit aromatischen Seitenketten eingeschoben ist;
R₁, R₂ und R₃ gleich oder unterschiedlich sind und jeweils für eine aromatische Aminosäure stehen, die aus Tyrosin, Tryptophan oder Phenylalanin ausgewählt wird;
X für eine beliebige Aminosäure steht, die innerhalb der Einheiten [R₁-(X)ₙ]_{w} und [R₂-(X)ₙ]₂ gleich oder unterschiedlich ist;
n 0 oder 1 ist;
w und z jeweils für eine natürliche Zahl von 0 bis 5 stehen;
und wobei zumindest eines von R₁, R₂ und R₃ so chemisch oder enzymatisch hydroxyliert wird, dass zumindest zwei Hydroxylgruppen am aromatischen Ring vorhanden sind;
(iii) den Aminosäuresequenzen - (Phe)ₙ-, -(Tyr)ₙ- oder - (Trp)ₙ- mit n=2-50 oder Kombinationen davon, wobei zumindest ein Phe, Tyr oder Trp so chemisch oder enzymatisch hydroxyliert wird, dass zumindest zwei Hydroxylgruppen am aromatischen Ring vorhanden sind; und
(iv) Analoga der unter (ii) und (iii) genannten Peptide, in denen die CO-NH-Amidbindungen durch eine oder mehrere der Gruppen, die aus Depsipeptiden (CO-O), Iminomethylenen (CH2-NH), trans-Alkenen (CH=CH), Enaminonitrilen (C(= CH-CN)-NH), Thioamiden (CS-NH), Thiomethylenen (S-CH2), Methylenen (CH2-CH2) und Retro-amiden (NH-CO) besteht, ausgewählt werden, substituiert ist;
besteht, ausgewählt wird.

2. Polypeptid nach Anspruch 1, bei dem zumindest eine aromatische Seitenkette des organischen Rests chemisch oder enzymatisch so hydroxyliert wird, dass zwei Hydroxylgruppen benachbart am aromatischen Ring vorhanden sind.

3. Polypeptid nach Anspruch 1, bei dem zumindest eine aromatische Seitenkette des organischen Rests chemisch oder enzymatisch so hydroxyliert wird, dass drei Hydroxylgruppen benachbart am aromatischen Ring vorhanden sind.

4. Polypeptid nach Anspruch 1, bei dem der organische Rest die Struktur P-[R₁-(X)ₙ]_{w}-[R₂-(X)ₙ]_{z}-R₃ besitzt, wobei P das bioaktive Polypeptid darstellt, das C-terminal oder N-terminal mit dem organischen Rest mit aromatischen Seitenketten verknüpft ist oder in das der organische Rest mit aromatischen Seitenketten eingeschoben ist; R₁, R₂ und R₃ gleich oder unterschiedlich sind und jeweils für eine aromatische Aminosäure stehen, die aus Tyrosin, Tryptophan oder Phenylalanin ausgewählt wird; mindestens eines von R₁, R₂ und R₃ für Tyrosin steht; X für eine beliebige Aminosäure steht, die innerhalb der Einheiten [R₁-(X)ₙ]_{w} und [R₂-(X)ₙ]_{z} gleich oder unterschiedlich ist; n 0 oder 1 ist; w und z jeweils für eine natürliche Zahl von 0 bis 5 stehen; wobei zumindest eines von R₁, R₂ und R₃ so chemisch oder enzymatisch hydroxyliert wird, dass zumindest zwei Hydroxylgruppen am aromatischen Ring vorhanden sind.

5. Polypeptid nach Anspruch 4, wobei durch die chemische oder enzymatische Hydroxylierung zumindest zwei 3,4-Dihydroxyphenylalanin(DOPA)-Reste gebildet werden.

6. Polypeptid nach Anspruch 1, bei dem der organische Rest mit aromatischen Seitenketten die Aminosäuresequenz -(Phe)ᵤ-, -(Tyr)ₙ- oder -(Trp)ₙ- mit n=2-50 ist, die in Form eines Fusionsproteins C-terminal oder N-terminal mit dem bioaktiven Polypeptid verknüpft ist oder in das bioaktive Polypeptid eingeschoben ist, wobei zumindest ein Phe, Tyr oder Trp so chemisch oder enzymatisch hydroxyliert wird, dass zumindest zwei Hydroxylgruppen am aromatischen Ring vorhanden sind.

7. Polypeptid nach Anspruch 6, bei dem der organische Rest mit dem bioaktiven Polypeptid am C- oder N-Terminus fusioniert ist.

8. Polypeptid nach einem der Ansprüche 6 bis 7, wobei der organische Rest mit aromatischen Seitenketten -(Tyr)ₙ- mit n=2-50 ist, wobei -(Tyr)ₙ- chemisch oder enzymatisch so hydroxyliert wird, dass -(DOPA)ₙ- entsteht.

9. Polypeptid nach einem der Ansprüche 6-8, wobei n zwei bis fünf ist.

10. Polypeptid nach einem der vorhergehenden Ansprüche, bei dem das bioaktive Polypeptid ein Wachstumsfaktor ist.

11. Polypeptid nach Anspruch 10, bei dem der Wachstumsfaktor ausgewählt wird aus der Gruppe, die aus Wachstumsfaktoren der TGF-β-Superfamilie, Knochenwachstumsfaktoren der BMP-Familie, knorpelbildenden Faktoren, und Blutgefäßwachstumsfaktoren sowie den Nell-Proteinen besteht.

12. Polypeptid nach Anspruch 1, bei dem die Synthese des organischen Rests auf chemischem Wege erfolgt und dieser organische Rest an einem Aminosäurerest des bioaktiven Polypeptids kovalent gebunden wird.

13. Polypeptid nach Anspruch 1, bei das mit einem organischen Rest modifizierte Polypeptid gentechnisch in Pro- oder Eukaryontenzellen synthetisiert wird.

14. Verfahren zur Beschichtung eines Substrates, bei dem eine Lösung eines Polypeptids gemäß einem der Ansprüche 1-13 auf die Oberfläche eines Substrates aufgebracht wird und das Polypeptid mittels kovalenter oder nicht-kovalenter Wechselwirkungen auf der Substratoberfläche immobilisiert wird.

15. Verfahren nach Anspruch 14, bei dem das Substrat Metall, Keramik oder Glas ist.

16. Verfahren nach Anspruch 15, bei dem das Substrat eine Oxy- oder Hydroxygruppen tragende Oberfläche aus Metalloxid, Metallhydroxid, Calciumhydroxyphosphaten (Hydroxyapatit), Siliciumoxid oder -hydroxid besitzt.

17. Verfahren nach Anspruch 14, 15 oder 16, bei dem das Substrat in Form eines Implantates für Tier oder Mensch eingesetzt wird.

18. Verfahren nach Anspruch 17, bei dem ein Polypeptid gemäß einem der Ansprüche 1-13 in Form eines mit einem Poly-DOPA- oder Poly-TOPA-Tag verbundenen Knochenwachstumsfaktors BMP-2 oder BMP-7 zur Beschichtung des Implantates eingesetzt wird.

19. Implantat, erhältlich nach einem der Verfahren gemäß Anspruch 14 bis 18.

20. Verwendung des Polypeptids gemäß einem der Ansprüche 1-13 in einem analytischen Verfahren zum Nachweis von Immunglobulinen oder Zellrezeptoren.

21. Verwendung des Polypeptids gemäß einem der Ansprüche 1-13 in einem affinitätschromatographischen Verfahren.

22. Ein mit einem organischen Rest modifiziertes Polypeptid, **dadurch gekennzeichnet, dass**
das modifizierte Polypeptid aus einem bioaktiven Polypeptid, das kovalent mit einem organischen Rest, der eine Rückgrat-Struktur mit aromatischen Seitenketten umfasst, verbunden ist, gebildet wird,
wobei der organische Rest aus der Gruppe, die aus
(i) Polymeren von bis zu 50 Monomeren von Ethylen, Propylen, Ester-, Ether- oder Thioetherverbindungen, die als Seitenketten Phenyl- oder Naphtylgruppen oder Heterocyclen aufweisen, die zumindest eine Hydroxylgruppe am aromatischen Rest tragen;
(ii)den Aminosäuresequenzen -(Tyr)ₙ- oder -(Trp)ₙ- mit n=2-50 oder Kombinationen davon; und
(iii) Analoga der unter (ii) genannten Peptide, in denen die CO-NH-Amidbindungen durch eine oder mehrere der Gruppen, die aus Depsipeptiden (CO-O), Iminomethylenen (CH2-NH), trans-Alkenen (CH=CH), Enaminonitrilen (C(= CH-CN)-NH), Thioamiden (CS-NH), Thiomethylenen (S-CH2), Methylenen (CH2-CH2) und Retro-amiden (NH-CO) besteht, ausgewählt werden, substituiert sind;
besteht, ausgewählt wird.

23. Polypeptid nach Anspruch 22, bei dem der organische Rest die Aminosäuresequenz -(Tyr)ₙ- mit n=2-50 ist.

24. Polypeptid nach Anspruch 23, bei dem n=3-5 ist.

25. Verfahren zur Beschichtung eines Substrates, bei dem eine Lösung eines Polypeptids gemäß einem der Ansprüche 22-24 auf die Oberfläche eines Substrates aufgebracht wird und das Polypeptid mittels kovalenter oder nicht-kovalenter Wechselwirkungen auf der Substratoberfläche immobilisiert wird.

26. Implantat, erhältlich nach einem Verfahren gemäß Anspruch 25.

## Claims

1. A polypeptide modified with an organic moiety, **characterized in that**
the modified polypeptide is composed of a bioactive polypeptide which is covalently bound to an organic moiety comprising a backbone structure having aromatic side chains,
wherein the organic moiety comprising a backbone structure having aromatic side chains is selected from the group consisting of
(i) polymers of up to about 50 monomers of ethylene, propylene, ester-, ether- or thioether compounds having side chains which are phenyl- or naphthyl groups, heterocycles or aromatic amino acids, like Phe, Tyr or Trp, which carry at least one hydroxy group at the aromatic moiety, and wherein at least one aromatic side chain of the organic moiety is chemically or enzymatically hydroxylated in such a way that this aromatic side chain carries two hydroxy groups;
(ii) a moiety with the structure
P-[R₁-(X)ₙ]_{w}-[R₂-(X)ₙ]_{z}-R₃
wherein
P represents the bioactive polypeptide which is C-terminally or N-terminally coupled to the organic moiety having aromatic side chains or into which the organic moiety having aromatic side chains is inserted;
R₁, R₂ and R₃ are the same or different and each represent an aromatic amino acid which is selected from the group consisting of Tyrosine, Tryptophane or Phenylalanine;
X represents any amino acid which is the same or different within the units [R₁-(X)ₙ]_{w} and [R₂-(X)ₙ]_{z};
n is 0 or 1;
w and z represent an integer from 0 to 5;
and wherein at least one of R₁, R₂ and R₃ is chemically or enzymatically hydroxylated in such a way that at least two hydroxy groups are present at the aromatic ring;
(iii) the amino acid sequences -(Phe)ₙ-, -(Tyr)ₙ- or -(Trp)ₙ- with n=2-50 or combinations thereof, wherein at least one Phe, Tyr or Trp is chemically or enzymatically hydroxylated in such a way that at least two hydroxy groups are present at the aromatic ring; and
(iv) analogs of the peptides listed under (ii) and (iii), wherein the CO-NH-amid linkages are substituted by one or more groups selected from the group consisting of depsipeptides (CO-O), iminomethylenes (CH₂-NH), trans-alkenes (CH=CH), enaminonitriles (C(=CH-CN)-NH), thioamides (CS-NH), thiomethylenes (S-CH₂), methylenes (CH₂-CH₂) and retroamides (NH-CO).

2. The polypeptide according to claim 1, wherein at least one aromatic side chain of the organic moiety is chemically or enzymatically hydroxylated in such a way that two adjacent hydroxy groups are present at the aromatic ring.

3. The polypeptide according to claim 1, wherein at least one aromatic side chain of the organic moiety is chemically or enzymatically hydroxylated in such a way that three adjacent hydroxy groups are present at the aromatic ring.

4. The polypeptide according to claim 1, wherein the organic moiety has the structure P-[R₁-(X)ₙ]_{w}[R₂-(X)ₙ]_{z}-R₃, wherein P represents the bioactive polypeptide which is C-terminally or N-terminally coupled to the organic moiety having aromatic side chains or into which the organic moiety having aromatic side chains is inserted; R₁, R₂ and R₃ are the same or different and each represent an aromatic amino acid which is selected from the group consisting of Tyrosine, Tryptophane or Phenylalanine; at least one of R₁, R₂ and R₃ is Tyrosine; X represents any amino acid which can be the same or different within the units [R₁-(X)ₙ]_{w} and [R₂-(X)ₙ]_{z}; n is 0 or 1; w and z represent an integer from 0 to 5; and wherein at least one of R₁, R₂ and R₃ is chemically or enzymatically hydroxylated in such a way that at least two hydroxy groups are present at the aromatic ring.

5. The polypeptide according to claim 4, wherein at least two 3,4-dihydroxyphenylalanine(DOPA) residues are formed by the chemical or enzymatic hydroxylation.

6. The polypeptide according to claim 1, wherein the organic moiety having aromatic side chains is the amino acid sequence -(Phe)ₙ-, -(Tyr)ₙ- or -(Trp)ₙ- with n=2-50 which is C-terminally or N-terminally linked to the bioactive polypeptide in form of a fusion protein or is inserted into the bioactive polypeptide, wherein at least one of Phe, Tyr or Trp is chemically or enzymatically hydroxylated in such a way that at least two hydroxy groups are present at the aromatic ring.

7. The polypeptide according to claim 6, wherein the organic moiety is fused with the bioactive polypeptide at the C- or N-terminus.

8. The polypeptide according to any of claims 6 to 7, wherein the organic moiety having aromatic side chains is -(Tyr)ₙ with n=2-50, wherein -(Tyr)ₙ- is chemically or enzymatically hydroxylated in such a way that -(DOPA)ₙ- is formed.

9. The polypeptide according to any of claims 6-8, wherein n is two to five.

10. The polypeptide according to any of the preceding claims, wherein the bioactive polypeptide is a growth factor.

11. The polypeptide according to claim 10, wherein the growth factor is selected from the group consisting of growth factors of the TGF-β superfamily, bone growth factors of the BMP family, cartilage forming factors, blood vessel growth factors and nell proteins.

12. The polypeptide according to claim 1, wherein the synthesis of the organic moiety is carried out chemically and said organic moiety is covalently bound to an amino acid residue of the bioactive polypeptide.

13. The polypeptide according to claim 1, wherein the polypeptide modified with an organic moiety is genetically synthesized in prokaryotic or eukaryotic cells.

14. A method for coating a substrate, wherein a solution of a polypeptide according to any one of claims 1 to 13 is applied to a surface of a substrate and the polypeptide is immobilized on the surface of the substrate by covalent or non-covalent interactions.

15. The method according to claim 14, wherein the substrate is metal, ceramic or glass.

16. The method according to claim 15, wherein the substrate has an oxy or hydroxy group carrying surface made of metal oxide, metal hydroxide, calcium hydroxyphosphates (hydroxyapatite), silicon oxide or silicon hydroxide.

17. The method according to claim 14, 15 or 16, wherein the substrate is used in form of an implant for animals or humans.

18. The method according to claim 17, wherein a polypeptide according to any one of claims 1 to 13 in form of a bone growth factor BMP-2 or BMP-7 which is linked to a poly-DOPA- or poly-TOPA-tag is used for coating the implant.

19. An implant obtainable by any one of the methods according to claims 14 to 18.

20. Use of a polypeptide according to any one of claims 1-13 in an analytical method for the detection of immunoglobulins or cell receptors.

21. Use of the polypeptide according to any one of claims 1-13 in an affinity chromatography method.

22. A polypeptide modified with an organic moiety, **characterized in that**
the modified polypeptide is composed of a bioactive polypeptide which is covalently bound to an organic moiety comprising a backbone structure having aromatic side chains,
wherein the organic moiety is selected from the group consisting of
(i) polymers of up to about 50 monomers of ethylene, propylene, ester-, ether- or thioether compounds having side chains which are phenyl- or naphthyl groups, or heterocycles, carrying at least one hydroxy group at the aromatic moiety;
(ii) the amino acid sequences -(Tyr)ₙ- or -(Trp)ₙ- with n=2-50 or combinations thereof; and
(iii) analogs of the peptides listed under (ii), wherein the CO-NH-amid linkages are substituted by one or more groups selected from the group consisting of depsipeptides (CO-O), iminomethylenes (CH₂-NH), trans-alkenes (CH=CH), enaminonitriles (C(=CH-CN)-NH), thioamides (CS-NH), thiomethylenes (S-CH₂), methylenes (CH₂-CH₂) and retroamides (NH-CO).

23. The polypeptide according to claim 22, wherein the organic moiety is the amino acid sequence -(Tyr)ₙ- with n=2-50.

24. The polypeptide according to claim 23, wherein n=3-5.

25. A method for coating a substrate, wherein a solution of a polypeptide according to any one of claims 22 to 24 is applied to a surface of a substrate and the polypeptide is immobilized on the surface of the substrate by covalent or non-covalent interactions.

26. An implant obtainable by the method according to claim 25.

## Revendications

1. Polypeptide modifié par un radical organique, **caractérisé en ce que**
le polypeptide modifié est formé à partir d'un polypeptide bioactif qui est lié par liaison covalente à un radical organique qui comprend une structure de squelette à chaînes latérales aromatiques,
le radical organique qui comprend une structure de squelette à chaînes latérales aromatiques étant choisi dans le groupe qui consiste en
(i) des polymères de jusqu'à 50 monomères d'éthylène, propylène, composés ester, éther ou thioéther, qui comportent comme chaînes latérales des groupes phényle ou naphtyle, des hétérocycles ou des aminoacides aromatiques tels que Phe, Tyr ou Trp, qui comportent au moins un groupe hydroxy sur le radical aromatique et au moins une chaîne latérale aromatique du radical organique étant hydroxylée par voie chimique ou enzymatique de sorte que cette chaîne latérale porte deux groupes hydroxy ;
(ii) un radical ayant la structure
P-[R₁-(X)ₙ]_{w}-[R₂-(X)ₙ]_{z}-R₃
dans laquelle
P représente le polypeptide bioactif, qui est lié à l'extrémité C-terminale ou N-terminale au radical organique à chaînes latérales aromatiques ou est inséré dans le radical organique à chaînes latérales aromatiques ;
R₁, R₂ et R₃ sont identiques ou différents et représentent chacun un aminoacide aromatique qui est choisi parmi la tyrosine, le tryptophane et la phénylalanine ;
X représente un aminoacide quelconque, qui est identique ou différent dans les motifs [R₁-(X)ₙ]_{w} et [R₂(X)ₙ]_{z} ;
n est 0 ou 1 ;
w et z représentent chacun un nombre naturel allant de 0 à 5 ;
et dans laquelle au moins l'un des radicaux R₁, R₂ et R₃ est hydroxylé par voie chimique ou enzymatique de sorte qu'au moins deux groupes hydroxy sont présents sur le cycle aromatique ;
(iii) les séquences d'aminoacides -(Phe)ₙ-, -(Tyr)ₙ- ou -(Trp)ₙ où n = 2-50 ou des associations de celles-ci, au moins un aminoacide Phe, Tyr ou Trp étant hydroxylé par voie chimique ou enzymatique de sorte qu'au moins deux groupes hydroxy sont présents sur le cycle aromatique ; et
(iv) des analogues des peptides mentionnés en (ii) et (iii), dans lesquels les liaisons amide CO-NH sont substituées par un ou plusieurs des groupes choisis parmi des depsipeptides (CO-O), iminométhylènes (CH₂-NH), *trans-*alcènes (CH=CH), énaminonitriles [C(=CH-CN)-NH], thioamides (CS-NH), thiométhylènes (S-CH₂), méthylènes (CH₂-CH₂) et rétroamides (NH-CO).

2. Polypeptide selon la revendication 1, dans lequel au moins une chaîne latérale aromatique du radical organique est hydroxylée par voie chimique ou enzymatique de sorte que deux groupes hydroxy sont présents en étant contigus sur le cycle aromatique.

3. Polypeptide selon la revendication 1, dans lequel au moins une chaîne latérale aromatique du radical organique est hydroxylée par voie chimique ou enzymatique de sorte que trois groupes hydroxy sont présents en étant contigus sur le cycle aromatique.

4. Polypeptide selon la revendication 1, dans lequel le radical organique a la structure P-[R₁₋(X)ₙ]_{w}-[R₂-(X)ₙ]_{z}-R₃, dans laquelle P représente le polypeptide bioactif, qui est lié à l'extrémité C-terminale ou N-terminale au radical organique à chaînes latérales aromatiques ou est inséré dans le radical organique à chaînes latérales aromatiques ; R₁, R₂ et R₃ sont identiques ou différents et représentent chacun un aminoacide aromatique qui est choisi parmi la tyrosine, le tryptophane et la phénylalanine ; au moins l'un des radicaux R₁, R₂ et R₃ représente la tyrosine ; X représente un aminoacide quelconque qui est identique ou différent dans les motifs [R₁-(X)ₙ]_{w} et [R₂-(X)ₙ]_{z} ; n est 0 ou 1 ; w et z représentent chacun un nombre naturel allant de 0 à 5 ; au moins l'un des radicaux R₁, R₂ et R₃ étant hydroxylé par voie chimique ou enzymatique de sorte qu'au moins deux groupes hydroxy sont présents sur le cycle aromatique.

5. Polypeptide selon la revendication 4, dans lequel au moins deux radicaux 3,4-dihydrophénylalanine (DOPA) sont formés par hydroxylation chimique ou enzymatique.

6. Polypeptide selon la revendication 1, dans lequel le radical organique à chaînes latérales aromatiques est la séquence d'aminoacides -(Phe)ₙ-, -(Tyr)ₙ- ou -(Trp)ₙ-où n = 2-50, qui est liée, sous forme d'une protéine de fusion, à l'extrémité C-terminale ou N-terminale au polypeptide bioactif ou est insérée dans le polypeptide bioactif, au moins un aminoacide Phe, Tyr ou Trp étant hydroxylé par voie chimique ou enzymatique de sorte qu'au moins deux groupes hydroxy sont présents sur le cycle aromatique.

7. Polypeptide selon la revendication 6, dans lequel le radical organique est fusionné à l'extrémité C- ou N-terminale avec le polypeptide bioactif.

8. Polypeptide selon la revendication 6 ou 7, dans lequel le radical organique à chaînes latérales aromatiques est -(Tyr)ₙ- où n = 2-50, -(Tyr)ₙ étant hydroxylé par voie chimique ou enzymatique de sorte qu'il en résulte -(DOPA)ₙ-.

9. Polypeptide selon l'une quelconque des revendications 6 à 8, dans lequel n vade2à5.

10. Polypeptide selon l'une quelconque des revendications précédentes, dans lequel le polypeptide bioactif est un facteur de croissance.

11. Polypeptide selon la revendication 10, dans lequel le facteur de croissance est choisi dans le groupe qui consiste en les facteurs de croissance de la superfamille TGF-β, les facteurs de croissance osseuse de la famille BMP, les facteurs chondrogéniques et les facteurs angiogéniques ainsi que les protéines Nell.

12. Polypeptide selon la revendication 1, dans lequel la synthèse du radical organique s'effectue par voie chimique et ce radical organique est lié par liaison covalente à un résidu aminoacide du polypeptide bioactif.

13. Polypeptide selon la revendication 1, dans lequel le polypeptide modifié par un radical organique est synthétisé par génie génétique dans des cellules procaryotes ou eucaryotes.

14. Procédé pour le revêtement d'un support, dans lequel une solution d'un polypeptide selon l'une quelconque des revendications 1 à 13 est appliquée sur la surface d'un support et le polypeptide est immobilisé par des interactions covalentes ou non covalentes sur la surface du support.

15. Procédé selon la revendication 14, dans lequel le support est un métal, une céramique ou du verre.

16. Procédé selon la revendication 15, dans lequel le support a une surface en oxyde métallique, hydroxyde métallique, hydroxyphosphates de calcium (hydroxyapatite), oxyde ou hydroxyde de silicium, portant des groupes oxy ou hydroxy.

17. Procédé selon la revendication 14, 15 ou 16, dans lequel le support est utilisé sous forme d'un implant pour l'homme ou l'animal.

18. Procédé selon la revendication 17, dans lequel on utilise pour le revêtement de l'implant un polypeptide selon l'une quelconque des revendications 1 à 13, sous forme d'un facteur de croissance osseuse BMP-2 ou BMP-7 lié à une étiquette Poly-DOPA- ou Poly-TOPA-Tag.

19. Implant pouvant être obtenu conformément à l'un des procédés selon les revendications 14 à 18.

20. Utilisation du polypeptide selon l'une quelconque des revendications 1 à 13, dans un procédé analytique pour la détection d'immunoglobulines ou de récepteurs cellulaires.

21. Utilisation du polypeptide selon l'une quelconque des revendications 1 à 13, dans un procédé de chromatographie par affinité.

22. Polypeptide modifié par un radical organique, **caractérisé en ce que**
le polypeptide modifié est formé à partir d'un polypeptide bioactif qui est lié par liaison covalente à un radical organique qui comprend une structure de squelette à chaînes latérales aromatiques,
le radical organique étant choisi dans le groupe qui consiste en
(i) des polymères de jusqu'à 50 monomères d'éthylène, propylène, composés ester, éther ou thioéther, qui comportent comme chaînes latérales des groupes phényle ou naphtyle ou des hétérocycles, qui comportent au moins un groupe hydroxy sur le radical aromatique ;
(ii) les séquences d'aminoacides-(Tyr)ₙ- ou -(Trp)ₙ- où n = 2-50 ou des associations de celles-ci ; et
(iii) des analogues des peptides mentionnés en (ii), dans lesquels les liaisons amide CO-NH sont substituées par un ou plusieurs des groupes choisis parmi des depsipeptides (CO-O), iminométhylènes (CH₂-NH), *trans*-alcènes (CH=CH), énaminonitriles [C(=CH-CN)-NH], thioamides (CS-NH), thiométhylènes (S-CH₂), méthylènes (CH₂-CH₂) et rétroamides (NH-CO).

23. Polypeptide selon la revendication 22, dans lequel le radical organique est la séquence d'aminoacides -(Tyr)ₙ- où n = 2-50.

24. Polypeptide selon la revendication 23, dans lequel n = 3-5.

25. Procédé pour le revêtement d'un support, dans lequel une solution d'un polypeptide selon l'une quelconque des revendications 22 à 24 est appliquée sur la surface d'un support et le polypeptide est immobilisé par des interactions covalentes ou non covalentes sur la surface du support.

26. Implant pouvant être obtenu conformément à un procédé selon la revendication 25.
